# EUROPÄISCHE PATENTANMELDUNG

(11) **EP 1 070 481 A2**
(43) Veröffentlichungstag der Anmeldung: **24.01.2001**
(21) Anmeldenummer: 00202412.3
(22) Anmeldetag: 07.07.2000
(51) Int. Cl.: A61B 6/03

(54) **Computertomograph mit kegelförmigem Strahlenbündel und helixförmiger Abtastbahn**

(30) Priorität: 17.07.1999 DE 19933649
(71) Anmelder: Philips Corporate Intellectual Property GmbH, 52064 Aachen (DE); Koninklijke Philips Electronics N.V., 5621 BA Eindhoven (NL)
(72) Erfinder: Proksa, Roland, Philips Corporate, 52064 Aachen (DE)
(74) Vertreter: Volmer, Georg, Dipl.-Ing.

(57) **Zusammenfassung**

Bei Computertomographen mit kegelförmigen Stahlenbündel und helixförmiger Abtastbahn werden die verschiedenen Voxel im Untersuchungsbereich - abhängig von ihrer Lage - unterschiedlich lange bestrahlt. Daraus ergibt sich ein örtlich inhomogenes Signal/Rausch-Verhältnis.

Um eine örtlich homogenes Signal/Rausch-Verhältnis zu erreichen, wird zwischen der Strahlenquelle und dem Objekt ein Filter angeordnet, das die Strahlung ortsabhängig in der Weise dämpft, daß der Rauschpegel aller Voxel zumindest näherungsweise gleich ist.

## Beschreibung

Die Erfindung betrifft einen Computertomographen mit
- einer Abtasteinheit, die eine Strahlenquelle und eine damit verbundene Detektoreinheit zur Erfassung eines von der Strahlenquelle emittierten, kegelförmigen Strahlenbündels nach dem Durchgang durch einen Untersuchungsbereich bzw. durch ein darin befindliches Objekt umfaßt
- einer Antriebsanordnung zur Erzeugung einer eine Rotation um eine Rotationsachse und einen Vorschub parallel zur Rotationachse umfasssenden Relativbewegung in Form einer Helix zwischen der Abtasteinheit und dem Untersuchungsbereich bzw. dem Objekt
und mit einer Rekonstruktionseinheit zur Rekonstruktion der räumlichen Verteilung der Absorption innerhalb des Untersuchungsbereiches aus den von der Detektoreinheit innerhalb eines durch die Helix definierten Detektorfensters akquirierten Meßdaten.

Ein solcher Comutertomograph ist aus der PCT-Anmeldung SE 98/00029 vom 14. Januar 98 bekannt. Bei dem bekannten Computertomographen werden zur Rekonstruktion der Absorptionsverteilung nur diejenigen Meßdaten herangezogen, die sich innerhalb eines Detektotfensters (als Detektorfenster wird hierbei und im folgenden der Teil der Messfläche der Detektoreinheit bezeichnet, der die für die Rekonstruktion herangezogenen Meßdaten - und nur diese - erfaßt) befinden, das in Richtung der z-Achse durch die Projektion zweier aufeinanderfolgenden Windungen der Helix definiert ist. Es läßt sich zeigen, daß bei einer solchen Gestaltung des Detektorfensters jedes Voxel im Untersuchungsbereich bei seinem Eintritt und bei seinem Austritt aus dem Strahlenbündel aus um exakt 180° versetzten Positionen (bezogen auf das jeweilige Voxel) auf das Detektorfenster projiziert wird. Die auf diese Weise erhaltenen Meßdaten erlauben eine einwandfreie Rekonstruktion der Absorptionsverteilung im Untersuchungsbereich, und zwar auch dann, wenn das darin enthaltene Objekt länger ist als der vom kegelförmigen Strahlenbündel erfaßte Teil des Untersuchungsbereichs.

Es sind auch noch andere Verfahren bekannt, bei denen pro Voxel nur Meßdaten aus einem Winkelbereich von exakt 180° herangezogen werden. Allerdings wird dabei die Absorptionsverteilung aus den Meßdaten auf andere Weise rekonstruiert.

Ein gewisser Nachteil der bekannten Verfahren besteht darin, daß sich das Rauschen nicht gleichmäßig über das rekonstruierte 3D-Volumen verteilt. Diese im Bild erkennbare räumliche Inhomogenitat des Signal/Rauschverhältnisses beeinträchtigt die Bildqualität.

Aufgabe der vorliegenden Erfindung ist es, die Bildqualität weiter zu verbessern. Ausgehend von einem Computertomographen der eingangs genannten Art wird diese Aufgabe erfindungsgemäß dadurch gelöst, daß zwischen der Strahlenquelle und dem Untersuchungsbereich ein Filter vorgesehen ist, das einen solchen ortsabhängigen Dämpfungsverlauf hat, daß das Integral über die Dämpfung der Strahlung entlang einer von der Projektion eines Voxels auf das Detektorfenster definierten Trajektorie für die verschiedenen Voxel im Untersuchungsbereich wenigstens näherungsweise gleich groß ist.

Die Erfindung basiert zunächst auf der Erkenntnis, daß die Voxel im Untersuchungsbereich unterschiedlich lange im Strahlengang verweilen müssen, bis Meßdaten aus einem Winkelbereich von 180° (gemessen von dem betreffenden Voxel aus) akquiriert sind. Je geringer die Verweilzeit ist, desto schlechter wird das Signal/Rauschverhältnis, mit dem das betreffende Voxel rekonstruierbar ist.

Die Erfindung basiert auf der Überlegung, daß das Signal/Rauschverhältnis dadurch räumlich vergleichmäßigt werden kann, daß zwischen der Strahlenquelle und dem Untersuchungsbereich ein Filter vorgesehen wird, das einen solchen ortsabhängigen Dämpfungsverlauf hat, daß es die Strahlen für diejenigen Voxel, die länger im Strahlenbündel verweilen müssen, um die 180° Bedingung zu erfüllen, stärker schwächt als für Voxel mit einer kürzeren Verweildauer.

Die Erfindung basiert weiterhin auf der Erkenntnis, daß die Projektion des Voxels auf die Detektoreinheit (bzw. das darin befindliche, eingangs definierte Detektorfenster) auf der Detektoreinheit bzw. dem Detektorfenster eine Bahn ― im folgenden als Trajektorie bezeichnet ― beschreibt, wobei die Trajektorien für verschiedene Voxel voneinander verschieden sind. Deshalb ist es möglich (und nötig) den ortsabhängigen Dämpfungsverlauf so zu gestalten, daß das zeitliche Integral über die Intensität aller Strahlen, aus deren Messdaten ein Voxel rekonstruiert wird, für Voxel in verschiedenen Teilen des Untersuchungsbereichs zumindest annähernd gleich groß ist. Dies ist gleichbedeutend damit, daß die (Weg-) Integrale über die Dämpfung des Filters entlang der Trajektorien für die verschiedenen Trajektorien bzw. Voxel wenigstens näherungsweise gleich groß sind. Wenn diese Bedingungen erfüllt sind, ist das Signal/Rauschverhältnis, mit dem ein Voxel rekonstruiert werden kann, nicht mehr von der Verweildauer dieses Voxels im Strahlengang abhängig.

Filter zwischen der Strahlenquelle und dem Untersuchungsbereich sind bei Computertomographen zwar schon bekannt, jedoch keine solchen, die die mit der unterschiedlichen Verweildauer verschiedener Voxel im Strahlengang einhergehenden Effekte kompensierern können.

Eine weitere Ausgestaltung der Erfindung ist in Anspruch 2 angegeben. Während bei den bekannten Computertomographen die beiden Ränder des Detektorfensters voneinander einen Abstand haben, der der Projektion zweier benachbarter Windungen der Helix auf die Detektoreinheit entspricht, beträgt bei der Ausgestaltung nach Anspruch 2 dieser Abstand ein ungeradzahliges Vielfaches davon. Bei einem solchen Detektorfenster "sieht" jedes Voxel, das das kegelförmige Strahlenbündel passiert, die Strahlenquelle unter einem Winkelbereich von genau (2n+1)180°, mit n ≥1. Auch in diesem Fall ergeben sich keine redundanten Meßdaten, und alle innerhalb des Detektorfensters akquirierten Meßdaten können zur Rekonstruktion herangezogen werden. Auch wenn dabei das Singal/Rauschverhältnis nicht so stark ortsabhängig ist wie bei dem eingangs genannten Computertomographie, ergibt sich immer noch eine gewisse Schwankung, die sich durch die Anwendung des erfindungsgemäßen Filters vergleichgmäßigen läßt.

Die Abwicklung eines Detektorfensters, innerhalb dessen die für die Erfüllung der 180°-Bedingung erforderlichen Daten akquiriert werden können, hat eine von der Rechteckform abweichende Form. Es wäre jedoch nicht sinnvoll, einen Detektor zu verwenden, dessen wirksame Meßfläche exakt mit diesem Detektorfenster übereinstimmt, weil solche Formen unüblich sind und für CT-Untersuchungen mit einer kreisförmigen Bahn (anstelle einer helixförmigen Bahn) ungeeignet wären. In der Praxis wird man deshalb immer einen Detektor mit rechteckiger Meßfläche verwenden. Die in Anspruch 3 angegebene Weiterbildung sorgt nun dafür, daß nur die wirklich benötigten Daten akquiriert werden können und daß von dem im Untersuchungsbereich befindliche Patienten unnötige Strahlung ferngehalten wird

Bei CT-Untersuchungen mit kreisförmiger Abtastbahn, bei denen die Abtasteinheit und der Untersuchungsbereich sich nicht in Richtung der Rotationsachse verschieben, ist die Verwendung des erfindungsgemäßen Filters nicht sinnvoll. Dem trägt die in Anspruch 4 angegebene Ausgestaltung Rechnung.

Die Erfindung wird nachstehend anhand der Zeichnungen näher erläutert.
Es zeigen:
- Fig. 1: einen erfindungsgemäßen Computertomographen in schematischer Darstellung.
- Fig. 2: eine vereinfachte Draufsicht auf einen solchen Computertomographen.
- Fig. 3: Die sich dabei ergebenden geometrischen Verhältnisse.
- Fig. 4: Die Abwicklung eines Detektorfensters.
- Fig. 5: Eine Draufsicht auf das erfindungsgemäße Filter mit Linien gleicher Dämpfung und
- Fig. 6: die helixförmige Abtastbahn, die die Abtasteinheit und ein im Untersuchungsbereich befindliches Objekt bei einer Ausführungsform beschreiben.

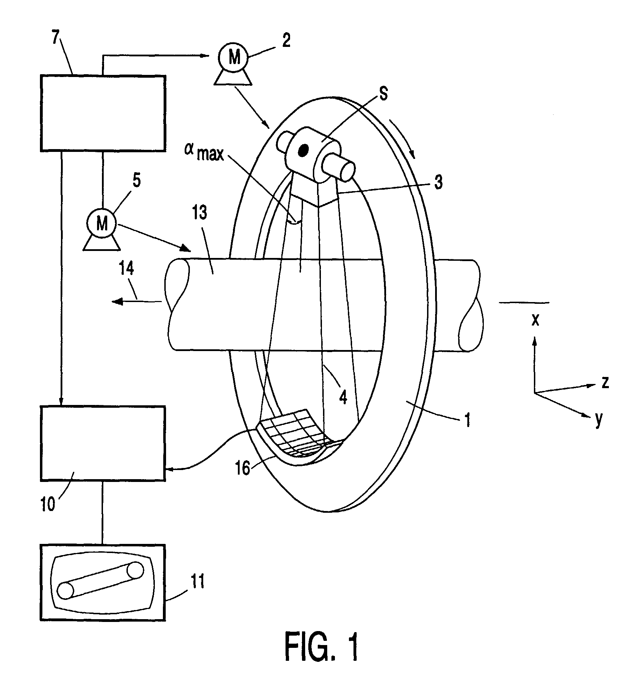

Der in Fig. 1 dargestellte Computertomograph umfaßt eine Gantry 1, die um eine parallel zur z-Richtung verlaufende Rotationsachse 14 rotieren kann. Dazu wird die Gantry von einem Motor 2 mit einer vorzugsweise konstanten, aber einstellbaren Winkelgeschwindigkeit angetrieben. An der Gantry ist eine Strahlenquelle S, beispielsweise ein Röntgenstrahler befestigt. Dieser ist mit einer Kollimatoranordnung 3 versehen, die aus der von der Strahlenquelle S erzeugten Quelle ein kegelförmiges Strahlenbündel 4 ausblendet, d.h. ein Strahlenbündel, das sowohl in Richtung der z-Achse als auch in einer dazu senkrechten Richtung (d.h. in der x-,y-Ebene des in Fig. 1 dargestellten Koordinatensystems) eine von Null verschiedene, endliche Ausdehnung hat.

Der in Fig. 1 dargestellte Computertomograph umfaßt eine Gantry 1, die um eine parallel zur z-Richtung verlaufende Rotationsachse 14 rotieren kann. Dazu wird die Gantry von einem Motor 2 mit einer vorzugsweise konstanten, aber einstellbaren Winkelgeschwindigkeit angetrieben. An der Gantry ist eine Strahlenquelle S, beispielsweise ein Röntgenstrahler befestigt. Dieser ist mit einer Kollimatoranordnung 3 versehen, die aus der von der Strahlenquelle S erzeugten Quelle ein kegelförmiges Strahlenbündel 4 ausblendet, d.h. ein Strahlenbündel, das sowohl in Richtung der z-Achse als auch in einer dazu senkrechten Richtung (d.h. in der x-,y-Ebene des in Fig. 1 dargestellten Koordinatensystems) eine von Null verschiedene, endliche Ausdehnung hat.

Der in Fig. 1 dargestellte Computertomograph umfaßt eine Gantry 1, die um eine parallel zur z-Richtung verlaufende Rotationsachse 14 rotieren kann. Dazu wird die Gantry von einem Motor 2 mit einer vorzugsweise konstanten, aber einstellbaren Winkelgeschwindigkeit angetrieben. An der Gantry ist eine Strahlenquelle S, beispielsweise ein Röntgenstrahler befestigt. Dieser ist mit einer Kollimatoranordnung 3 versehen, die aus der von der Strahlenquelle S erzeugten Quelle ein kegelförmiges Strahlenbündel 4 ausblendet, d.h. ein Strahlenbündel, das sowohl in Richtung der z-Achse als auch in einer dazu senkrechten Richtung (d.h. in der x-,y-Ebene des in Fig. 1 dargestellten Koordinatensystems) eine von Null verschiedene, endliche Ausdehnung hat.

Die von der Detektoreinheit 16 akquirierten Meßdaten werden einem Bildverarbeitungsrechner 10 zugeführt, der daraus die Absorptionsverteilung in dem vom Strahlenkegel 4 erfaßten Teil des Untersuchungsbereichs 13 rekonstruiert und z.B. auf einem Monitor wiedergibt. Die beiden Motoren 2 und 5, der Bildverarbeitungsrechner 10, die Strahlenquelle S und der Transfer der Meßdaten von der Detektoreinheit 16 zum Bildverarbeitungsrechner 10 werden von einer geeigneten Kontrolleinheit 7 gesteuert.

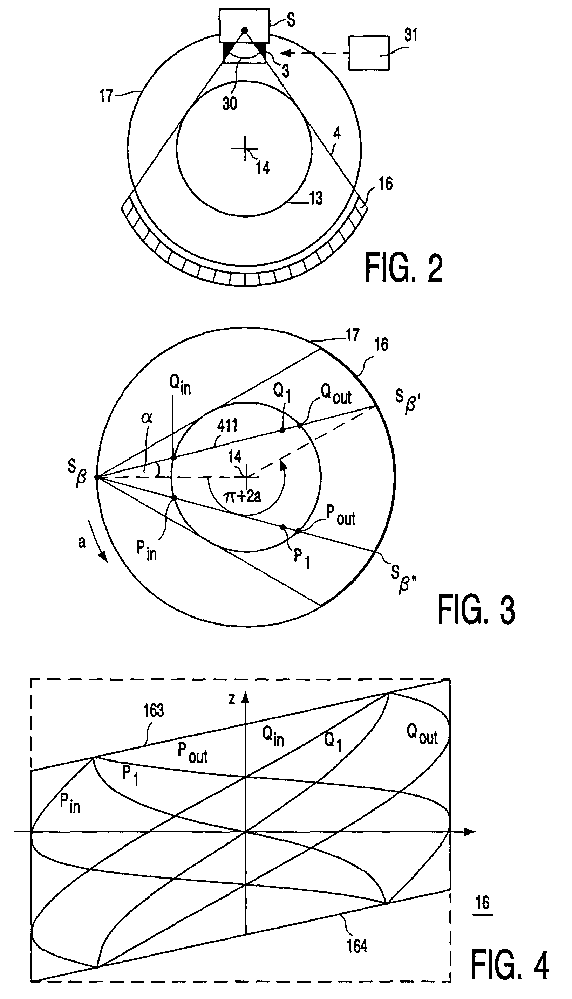

Fig. 2 stellt die Anordnung rein schematisch in einer Ansicht parallel zur z-Achse bzw. Rotationsachse 14 dar. Die Helix 17 geht dann in einen Kreis über und die Rotationsachse 14 in einen Punkt, den Mittelpunkt dieses Kreises. Das von der Strahlenquelle S emittierte Strahlenbündel 4 durchsetzt zunächst ein in dem Gehäuse des Kollimators 3 befindliches Filter 30 dessen Dicke ortsabhängig ist, bevor es in den Untersuchungsbereich eintritt.

Eine von der Steuereinheit 7 (Fig. 1) gesteuerte Filterwechseleinrichtung 31 gestattet es, das Filter 30 aus dem Strahlengang zu entfernen, beispielsweise um CT-Untersuchungen mit einer rein kreisförmigen Abtastbewegung ausfuhren zu können.

Fig. 3 stellt die geometrischen Verhältnisse in der gleichen Ansicht dar wie Fig. 2. Die Strahlenquelle S bewegt sich auf der helixförmigen Bahn 17 im Gegenuhrzeigersinn, d.h. in Richtung des Pfeiles a. Mit 411 ist ein Strahl aus dem Strahlenbündel 4 bezeichnet, der von der Strahlenquellenposition S_{β} ausgeht und ein Voxel Q₁ in dem Moment durchstrahlt, in dem es in den Strahlenkegel eintritt. Nachdem sich die Strahlenquelle aus ihrer Position S_{β} um den Winkel ϕ+2α (bezogen auf die Rotationsachse 14) gedreht hat, befindet sie sich in einer Position S_{β'} die bezüglich des Voxels Q₁ genau um den Winkel π (gegenüber der anfänglichen Position S_{β} der Strahlenquelle) gedreht ist und in der dieses Voxel gerade das Strahlenbündel 4 verläßt.

Andererseits ist ein Voxel P₁ dargestellt, bei dessen Eintritt in das Strahlenbündel sich die Strahlenquelle ebenfalls in der Position S_{β} befindet, bei dessen Austritt sie jedoch die Position S_{β''} einnimmt. Auch hierbei hat sich die Strahlenquelle um das Voxel P₁ um exakt den Winkel π gedreht ― um die Rotationsachse 14 jedoch nur um den Winkel π-2α.

Das Voxel Q₁ verweilt daher proportional zum Drehwinkel (π+2α) um die Rotationsachse länger als das Voxel P₁ (mit dem Drehwinkel π-2α) in dem kegelförmigen Strahlenbündel 4. Das Verhältnis dieser Verweildauern, das mit den Signal/Rauschverhältnis für die betreffenden Voxel korreliert ist, ist im ungünstigsten Fall (für αₘₐₓ = 30°)2:1, woraus sich eine starke Ortsabhängigkeit des Signal/Rauschverhältnisses ergibt.

In Fig. 3 sind außerdem die auf dem Strahl 411 an den beiden gegenüberliegenden Rändern des Untersuchungsbereiches 13 Liegenden Voxel Qᵢₙ und Qₒᵤₜ dargestellt. Weiterhin sind die Voxel Pᵢₙ und Pₒᵤₜ dargestellt, die beim Eintritt auf demselben Strahl liegen wie P₁ und die am vorderen und hinteren Rand des Untersuchungsbereichs liegen.

Ebenso sind die Trajektorien für die Voxel Pᵢₙ, P₁ und Pₒᵤₜ dargestellt. Diese schneiden sich wiederum in je einem Punkt am Anfang und am Ende, da sie beim Eintritt in und beim Austritt aus dem Strahlenbündel ebenfalls auf demselben Strahl liegen. Solche Trajektorien lassen sich noch für weitere Voxel darstellen, die jeweils beim Eintritt und beim Austritt auf der gleichen Geraden liegen, so daß sich die Trajektorien zu Beginn und zum Ende schneiden. Verbindet man alle diese Schnittpunkte einerseits beim Eintritt in das Strahlenbündel 4 und andererseits beim Austritt aus dem Strahlenbündel, dann erhält man die Randlinien 164 bzw. 163 des Detektorfensters. Es läßt sich zeigen, daß diese in z-Richtung gegeneinander versetzten Randlinien auf Verbindungsgeraden zwischen der Strahlenquelle und Punkten auf zwei aufeinanderfolgenden Windungen der Helix 17 liegen. In der Abwicklung sind diese Linien geradlinig, wenn das Detektorfenster auf einem Kreisbogen um die Rotationsachse 14 liegt, andernfalls sind sie gekrümmt.
Alle Meßdaten, die von Detektorelementen zwischen den beiden Randlinien (und den seitlichen vertikalen Linien, deren Abstand von der z-Achse durch den Öffnungswinkel αₘₐₓ des Strahlenbündels) bestimmt ist, werden für die Rekonstruktion benötigt. Die Meßdaten von Detektorelementen außerhalb dieser durchzogenen Linien ― also von von Detektorelementen in der linken oberen oder der rechten unteren Ecke des Detektors ― werden für die Rekonstruktion nicht benötigt. Sie sind für die Rekonstruktion irrelevant.

Die Verweildauer eines Voxels im Strahlengang hängt ― wie anhand der Fig. 3 erläutert, von der Lage dieses Voxels bei seinem Eintritt in das Strahlenbündel ab. Daraus läßt sich der räumliche Verlauf der Dämpfung des Filters 30 im Strahlengang zumindest näherungsweise ableiten: An den Stellen, an denen die Strahlen durch die relativ lange im Strahlenbündel befindlichen Voxel Q₁ , Qᵢₙ oder Qₒᵤₜ das Filter 30 passieren (die Bahnen, die diese Strahlen dabei auf dem Filter beschreiben, ähneln den Trajektorien), muß das Filter insgesamt eine relativ große Dämpfung haben. An den Stellen, an denen hingegen der Strahl zum Punkt P₁ das Filter passiert, muß es eine vergleichsweise niedrige Dämpfung haben, so daß das zeitliche Integral der Intensität (oder das Wegintegral der Dämpfung des Filters) für die Strahlen z.B. zu dem Voxel Q₁ einerseits und dem Voxel P₁ andererseits zumindest näherungsweise gleich groß ist.

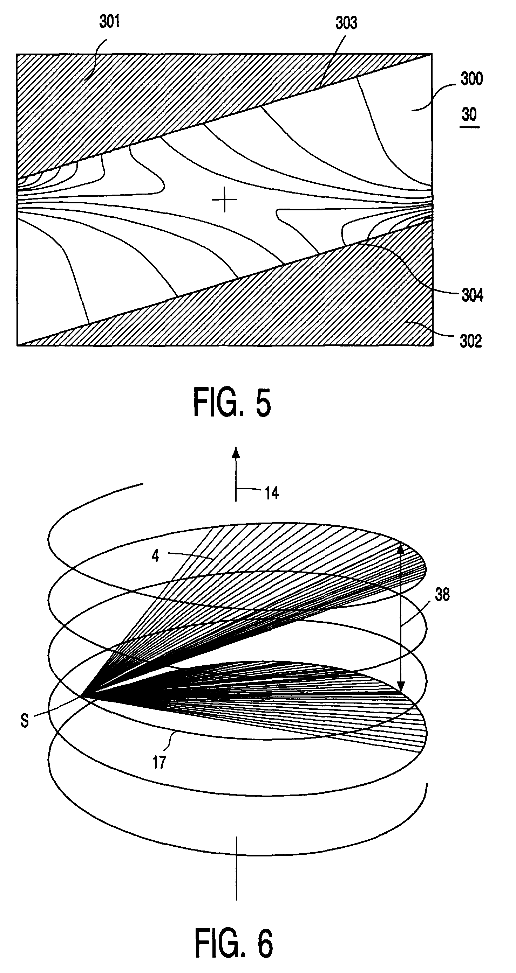

Fig. 5 stellt die Linien gleicher Dämpfung bei einer Abwicklung eines solchen Filter 30 dar. Die Linien sind symmetrisch bezüglich des Mittelpunktes. Die Dämpfung nimmt dabei von rechts Unten nach rechts oben und von links oben nach links Unten zu. Der raumliche Verlauf der Dämpfung bzw. der Dicke dieses Filters ist derart, daß der Strahl zu einem Voxel, dessen Projektion von links unten in Fig. 5 nach rechts oben verläuft hinter dem Filter etwa das gleiche zeitliche Integral aufweist wie ein Strahl, dessen Projektion von rechts unten nach links oben verläuft.

Die oberen und unteren Begrenzungstinien 303 und 304 dieses Filters haben einen entsprechenden Verlauf wie die Randlinien 164 und 163 des Detektorfensters, wenn das Verhältnis zwischen dem Abstand dieses Filters von der Strahlenquelle und dem Abstand der Detektoreinheit von der Strahlenquelle für aller von der Strahlenquelle ausgehenden Strahlen gleich ist. Oberhalb der Linie 303 und unterhalb der Linie 4 sind keilförmige Abschnitte 301 und 302 vorgesehen, die aus einem Material mit hoher Ordnungszahl (z. B. Blei) und ausreichender Dicke bestehen, um die dort auftreffende Strahlung praktisch vollständig zu absorbieren. Dadurch erhalten die außerhalb des durch die Randlinien 163 und 164 definierten Detektorfensters liegenden Detektorelemente des Detektors 16 keine Strahlung, und die Strahlenbelastung des Patienten wird reduziert.

Das Filter 30 besteht in seinem für die Röntgenstrahlung durchlässigen Teil 300 z.B. aus Aluminium und hat eine ortsabhängige Dicke, so daß sich der gewünschte räumliche Dämpfungsverlauf ergibt. Die Teile 300, 301 und 302 können auf einem gemeinsamen Träger aufgebracht sein, der die Röntgenstrahlung im wesentlichen nicht schwächt.

Bei dem zuvor beschriebenen Ausführungsbeispiel wurde davon ausgegangen, daß die Ränder des Detektorfensters und die Projektion zweier benachbarter Abschnitte der Helix zusammenfallen und daß der Vorschub in z-Richtung während einer vollen Umdrehung dem Abstand P dieser beiden Windungen in z-Richtung gemessen entspricht.

Es sind aber auch andere Geometrien möglich, von denen eine in Fig. 6 dargestellt ist. In der Zeichnung sind obere und untere Randstrahlen dargestellt, die das Detektorfenster definieren, innerhalb dessen die Meßwerte der Detektorelemente zur Rekonstruktion herangezogen werden. Man erkennt, daß auch diese Strahlen auf zwei Windungen der Helix treffen, allerdings besteht zwischen diesen Windungen der Abstand 3p (p ist der Abstand zwischen benachbarten Windungen). Wie in der deutschen Patentanmeldung......(PHD 98-086) beschrieben, "sieht" jedes Voxel, das in dieses Strahlenbündel ein- und wieder ausdringt die Strahlenquelle unter einem Winkel von 3π.
Die Abwicklung des zugehörigen Detektorfensters hat obere und untere Randlinien mit einer geringeren Steigung als das Detektorfenster gemäß Fig. 4 . Auch hierbei haben die Voxel des Untersuchungsbereichs eine Verweildauer im Strahlengang, die von der Lage dieses Voxels beim Eintritt (und Austritt) in das Strahlenbündel abhängt, doch ist die Abhängigkeit geringer als bei der zuvor beschriebenen Ausführungsform. Deshalb kann auch hier das erfindungsgemäße Filter ― mit einer weniger ausgeprägten Dämpfungscharakteristik als zuvor ― für ein weitgehend ortsunabhängiges Signal/Rauschverhäknis sorgen.

Ein erfindungsgemäßes Dämpfungsfilter ist immer dann anwendbar, wenn das Detektorfenster so beschaffen ist, daß die Strahlenquelle sich zwischen dem Eintritt eines Voxels in das kegelförmige Strahlenbündel und dem Austritt aus dem kegelförmigen Strahlenbündel um genau (2n+1)180° gedreht hat, wobei n eine ganze Zahl größer Null ist.

## Patentansprüche

1. Computertomograph mit
- einer Abtasteinheit, die eine Strahlenquelle (S) und eine damit verbundene Detektoreinheit (16) zur Erfassung eines von der Strahlenquelle emittierten, kegelförmigen Strahlenbündels nach dem Durchgang durch einen Untersuchungsbereich (13) bzw. durch ein darin befindliches Objekt umfaßt
- einer Antriebsanordnung (2,5) zur Erzeugung einer eine Rotation um eine Rotationsachse (14) und einen Vorschub parallel zur Rotationachse umfasssenden Relativbewegung in Form einer Helix zwischen der Abtasteinheit (S,16) und dem Untersuchungsbereich (13) bzw. dem Objekt
- und mit einer Rekonstruktionseinheit (10) zur Rekonstruktion der räumlichen Verteilung der Absorption innerhalb des Untersuchungsbereiches (13) aus den von der Detektoreinheit (16) innerhalb eines durch die Helix definierten Detektorfensters (160) akquirierten Meßdaten
dadurch gekennzeichnet,
daß zwischen der Strahlenquelle (S) und dem Untersuchungsbereich (13) ein Filter (30) vorgesehen ist, das einen solchen ortsabhängigen Dämpfungsverlauf hat, daß das Integral über die Dämpfung der Strahlung entlang einer von der Projektion eines Voxels auf das Detektorfenster definierten Trajektorie für die verschiedenen Voxel im Untersuchungsbereich wenigstens näherungsweise gleich groß ist.

2. Computertomograph nach Anspruch 1
dadurch gekennzeichnet,
daß die Verbindungsgeraden der Strahlenquelle (S) mit den beiden in Richtung der Rotationsachse (14) gegeneinander versetzten Rändern (163, 164) des Detektorfensters (160) zwei in Richtung der Rotationsachse um die Strecke (2n+1)p versetzte Windungen der Helix (17) schneiden, wobei n eine ganze Zahl ≥ 1 ist und p dem achsialen Versatz zweier benachbarter Windungen der Helix entspricht.

3. Computertomograph nach Anspruch 1
dadurch gekennzeichnet,
daß die Meßfläche der Detektoreinheit größer ist als das Detektorfenster und daß mit dem Filter (30) Blendenabschnitte (301, 302) verbunden sind, die die außerhalb des Detektorfensters befindlichen Teile der Detektoreinheit von Strahlung freihalten.

4. Computertomograph nach Anspruch 1
dadurch gekennzeichnet,
daß Mittel (31) zum Verschieben des Filters (30) aus dem Strahlengang vorgesehen sind.
